# EUROPEAN PATENT APPLICATION

(11) **EP 1 563 837 A1**
(43) Date of publication of application: **17.08.2005**
(21) Application number: 04002317.8
(22) Date of filing: 03.02.2004
(51) Int. Cl.: A61K 31/366, A61K 31/40, A61K 31/22, A61K 31/695, A61K 9/20, A61P 9/10

(54) **Hypocholesterolemic compositions comprising a statin and an antiflatulent agent**

(71) Applicant: FERRER INTERNACIONAL, S.A., 08028 Barcelona (ES)
(72) Inventor: Guerrero, Marta, 08024 Barcelona (ES); Orriols, Anna, 08024 Manresa (ES); Raga, Manuel M., 08024 Barcelona (ES); Guglietta, Antonio, 08970 Sant Joan Despi (ES)
(74) Representative: Reitstötter - Kinzebach & Partner (GbR)

(57) **Abstract**

The present invention relates to hypocholesterolemic compositions comprising statins plus antiflatulent agents. In particular, the compositions of the present invention comprise a statin selected from the group consisting of atorvastatin, cerivastatin, fluvastatin, lovastatin, pitavastatin, pravastatin, rosuvastatin and simvastatin, whether in free form or as pharmaceutically acceptable salts and hydrates thereof, plus an antiflatulent agent selected from the group consisting of simethicone and dimethicone. The combination of statins plus antiflatulent agents is useful in the prevention and management of flatulence caused by statins.

## Description

### Field of the invention

The present invention relates to hypocholesterolemic compositions comprising statins plus antiflatulent agents.

### Background of the invention

Statins are inhibitors of hydroxymethylglutaryl-CoA reductase, a key enzyme in the synthesis of cholesterol, which directly lower cholesterol levels. These compounds are known to be safe and effective hypocholesterolemic agents and they, therefore, represent an important therapeutic contribution to the treatment of coronary heart disease and to the reduction of morbidity and mortality by such serious cardiovascular pathological conditions.

Statins commonly used in medicine are atorvastatin (USP 5273995), cerivastatin (USP 5177080), fluvastatin (USP 4739073), lovastatin (USP 4231938), pravastatin (USP 4346227), rosuvastatin (USP RE 37314) and simvastatin (USP 4444784). They may be used in free form or as pharmarceutically acceptable salts thereof, generally alkaline or alkaline-earth salts, whether anhydrous or hydrated; it is usually desirable to use the sodium or calcium salts. For example, in clinical practice, atorvastatin is used as sodium (2:1) trihydrate salt, cerivastatin, fluvastatin and pravastatin as sodium salt, rosuvastatin as sodium salt and lovastatin and simvastatin in free form. A more recent compound, pitavastatin (EP 304063), is currently under Phase III development in Europe.

Among the most significant and frequent side effects of statins is flatulence (Bakker-Arkema *et al*, *Atherosclerosis* 2000 Mar, 149(1), 123-9 [PubMed 10704623]; Black *et al, Arch Intern Med* 1998 Mar 23, 158(6), 577-84 [PubMed 9521221]; Posvar *et al, J Clin Pharmacol* 1996 Aug, 36(8), 728-31 [PubMed 8877677]; Boccuzzi *et al, Am J Cardiol* 1991 Nov 1, 68(11), 1127-31 [Pubmed 1951069]; Zeller *et al, Drug Intell Clin Pharm* 1988 Jul-Aug, 22(7-8), 542-5 [PubMed 3046888]), which may be the cause of discomfort and symptomatological confusion, since its symptoms may be like those of coronary heart disease which is the aim of hypolipemic therapy by statins.

Among substances capable of decreasing flatulence are the antiflatulent agents having an antifoaming action. Simethicone and dimethicone, for instance, are successfully applied to the management of flatulence and meteorism. These compounds are effective if appropriate sanitary/dietetic measures are further applied, for example, avoidance of carbonated drinks and flatulent food.

Certain commercial products containing statins, like Lipitor (Atorvastatin as calcium 2:1 trihydrate), have already been formulated with an antifoaming agent, i.e. an emulsion of simethicone. However, the proportion of this compound is very low and it simply acts as a pharmaceutical carrier.

### Embodiment of the invention

The embodiment of the invention is to provide hypocholesterolemic compositions comprising a statin and an antiflatulent agent by antifoaming action in a proportion of active ingredient with the aim of relieving flatulence caused by the statin.

The combination of statins plus antiflatulent agents is useful in the prevention and management of flatulence caused by statins. This provides a better compliance of patients to the treatment and a better clinical understanding of symptoms, since both coronary heart diseases and flatulence are accompanied by thoracicoabdominal disturbances.

### Summary of the invention

The compositions of the present invention comprise a statin selected from the group consisting of atorvastatin, cerivastatin, fluvastatin, lovastatin, pitavastatin, pravastatin, rosuvastatin and simvastatin, whether in free form or as pharmaceutically acceptable salts and hydrates thereof, plus an antiflatulent agent selected from the group consisting of simethicone and dimethicone.

The compositions of the present invention may be administered orally in the form of tablets, capsules, coated tablets, syrups, solutions, powders, granules, emulsions or the like. The tablets and particularly the coated tablets are preferred.

The statins may be present in the tablets in an amount of 0.1 to 100 mg/tab. In turn, the antiflatulent agents may be present in the tablets in a proportion from 25 to 250 mg/tab.

The compositions of this invention further comprise other components selected from the group consisting of diluents, binders, disintegrants and lubricants, and mixtures thereof, which are commonly used in pharmaceutical technology. Other pharmaceutic excipients, like antioxidants and wetting agents, may be optionally added.

Due to the fact that statins are photosensitive it is convenient to protect the tablets with a coating comprising cellulose or acrylic derivatives, as well as plasticizers and opacifiers. Optionally, it is possible to add different colouring agents.

### Brief description of the drawing

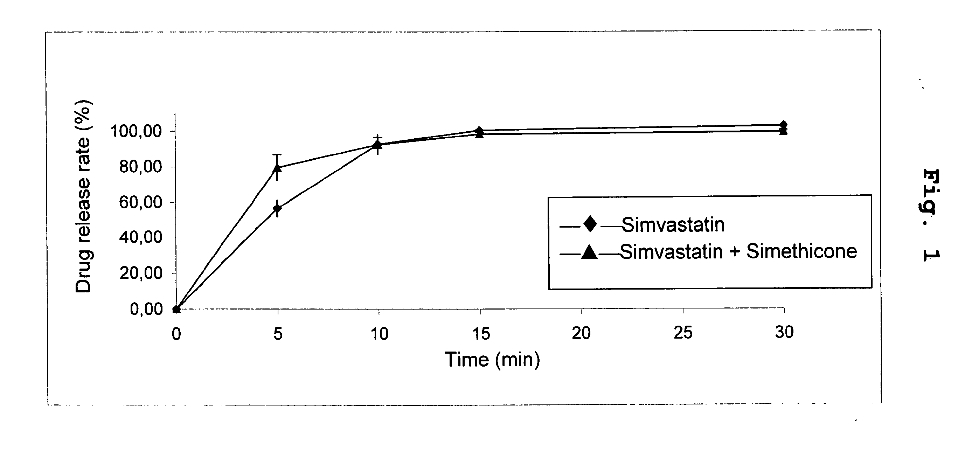

Figure 1 shows the *in vitro* dissolution profile, expressed in mean values, of the tablets of Example 4 comprising simvastatin plus simethicone, and other identical tablets without simethicone.

### Detailed description of the invention

The compositions of the present invention comprise a statin selected from the group consisting of atorvastatin, cerivastatin, fluvastatin, lovastatin, pitavastatin, pravastatin, rosuvastatin and simvastatin, whether in free form or as pharmaceutically acceptable salts and hydrates thereof, plus an antiflatulent agent selected from the group consisting of simethicone and dimethicone.

Preferably, atorvastatin is used as calcium (2:1) trihydrate, cerivastatin, fluvastatin and pravastatin as sodium salt, rosuvastatin as calcium salt and lovastatin and simvastatin in free form.

The compositions of the present invention may be administered orally in the form of tablets, capsules, coated tablets, syrups, solutions, powder, granules, emulsions or the like. The tablets and particularly the coated tablets are preferred.

Statins may be present in the tablets in an amount of 0.1 to 100 mg/tablet. Thus, in case of a standard tablet weighing 400 mg, the proportion of statin may range from 0.025 to 25%. Usually, the amounts of statin per tablet may be 0.1, 2.5, 5, 10, 20, 40 and 80 mg. Therefore, for a standard tablet of 400 mg, the proportion of statin may be 0.025%, 0.625%, 1.25%, 2.5%, 5%, 10% and 20% respectively.

In turn, the antiflatulent agents may be present in the tablets in an amount of 25 to 250 mg/tablet. Therefore, for a standard tablet of 400 mg, The proportion of the antiflatulent agent may range from 6.25 to 62.5%.

Preferred diluents in the tablets of the present invention are microcrystalline celluloses and derivatives thereof, for example Prosolv® which is a mixture of microcrystalline cellulose and colloidal silicon dioxide, lactose, mannitol, calcium phosphates, starch, and the like. Preferably, microcrystalline celluloses are Avicel® PH102 and Prosolv®.

Preferred binders in the tablets of the present invention are starch, polyethylene glycols, polyvinylpyrrolidone, cellulose derivatives, e.g., hydroxypropyl methylcellulose, and the like.

Preferred disintegrants in the tablets of the present invention are colloidal silicon dioxide, croscarmellose, polyvinylpyrrolidone, starch, and its pregenalitized derivatives, e.g., Primojel®, which is sodium starch glycolate, and the like. Preferably, Aerosil®, Acdisol® and polyvinylpyrrolidone are used.

Preferred lubricants in the tablets of the present invention are talc, magnesium stearate, stearic acid, sodium stearyl fumarate, high-molecular weight polyethylenglycol (4000 - 8000), e.g., PEG 8000, and the like. Preferably, sodium stearyl fumarate, talc and magnesium stearate are used.

Other pharmaceutic excipients, like antioxidants e.g., butylated hydroxyanisole, ascorbic acid or gluconolactone, and the like, and wetting agents e.g., sodium lauryl sulphate, and the like, may be optionally added.

The tablets of the present invention are provided with a light-resistant coating. Preferably, the coating consists of a layer constituted by cellulose derivatives, for example, sodium hydroxypropyl methylcellulose (HPMC), acrylic polymers, plasticizers, for example, diethyl citrate, opacifiers, for example titanium dioxide, talc and stearic acid. They may optionally contain pigments for tablet-colouring. As pigments, ferric oxide derivatives are preferred.

The method for preparing the statin core with the antiflatulent agents may be by precompression, i.e., a previous compaction of the mixture, followed by sieving and final compression. They may also be obtained by wet granulation using a hydroalcohol solvent. These are standard procedures in pharmaceutical technology.

However, the applicants have discovered that the tablets of the present invention may be prepared advantageously by direct compression, i.e., by directly compressing all the components. Thus, simethicone, which is liquid, is incorporated in the form of an adsorbate with an adsorbent substance, for example, Prosolv@, mannitol, anhydrous colloidal silica (silicon dioxide) or lactose. It is then sieved and mixed with the other components to yield the final mixture. The procedure of direct compression is preferred rather than usual precompression procedures because of its lower cost and easier scale-up manufacturing.

The solubility of the tablets of the present invention is not affected by the presence of an antiflatulent agent. Thus, Figure 1 shows that the dissolution profiles of the new tablets of Example 4, which contain the antiflatulent agent, namely simethicone, are not different from those of conventional tablets without antiflatulent agent. Consequently, there are no significant differences in the pharmaceutical behaviour of either preparation, in such a way that treatment of patients taking statins may be easily replaced with the tablets of the present invention.

The present invention is further illustrated by - but not limited to - the following examples.

### Example 1: 400 mg tablet containing 40 mg of atorvastatin (calcium trihydrate) and 115 mg of simethicone

| | |
|---|---|
| Atorvastatin (calcium trihydrate) | 40 mg |
| Simethicone | 115 mg |
| Anhydrous colloidal silica | 10 mg |
| Sodium croscarmellose | 10 mg |
| Sodium stearyl fumarate | 15 mg |
| Microcrystalline cellulose Avicel PH102 q.s. | 400 mg |

### Example 2: 400 mg tablet containing 20 mg of simvastatin and 125 mg of simethicone

| | |
|---|---|
| Simvastatin | 20 mg |
| Simethicone | 125 mg |
| Polyvinylpyrrolidone | 15 mg |
| Sodium croscarmellose | 5 mg |
| Sodium stearyl fumarate | 15 mg |
| Sodium lauryl sulfate | 4 mg |
| Butylated hydroxyanisole | 5 mg |
| Lactose q.s. | 400 mg |

### Example 3: 400 mg tablet containing 10 mg of sodium pravastatin and 125 mg of simethicone

| | |
|---|---|
| Sodium pravastatin | 10 mg |
| Simethicone | 125 mg |
| Primojel® | 20 mg |
| Talc | 12 mg |
| Magnesium stearate | 4 mg |
| Prosolv® q.s. | 400 mg |

### Example 4: 400 mg tablet containing 40 mg of simvastatin and 125 mg of simethicone

| | |
|---|---|
| Simvastatin | 40 mg |
| Simethicone | 125 mg |
| Primojel® | 16 mg |
| Silicon dioxide | 43 mg |
| Talc | 12 mg |
| Magnesium stearate | 4 mg |
| Lactose (direct compression) q.s. | 400 mg |

## Claims

1. A pharmaceutical composition comprising a statin and an antiflatulent agent in a suitable proportion as active ingredient.

2. A pharmaceutical composition according to claim 1 wherein the statin is selected from the group consisting of atorvastatin, cerivastatin, fluvastatin, lovastatin, pitavastatin, pravastatin, rosuvastatin and simvastatin, and pharmaceutically acceptable salts and hydrates thereof.

3. A pharmaceutical composition according to claim 2 wherein the statin is simvastatin or a pharmaceutically acceptable salt thereof.

4. A pharmaceutical composition according to any one of claims 1 to 3 wherein the antiflatulent agent is selected from the group consisiting of simethicone and dimethicone.

5. A pharmaceutical composition according to claim 4 wherein the antiflatulent agent is simethicone.

6. A pharmaceutical composition according to any one of the preceding claims wherein the composition is a tablet, capsule, coated tablet, syrup, solution, powder, granule, or emulsion.

7. A pharmaceutical composition according to claim 6 wherein the composition is a coated tablet.

8. A pharmaceutical composition according to claim 6 or 7 wherein simvastatin is present in an amount from 2.5 to 100 mg per tablet.

9. A pharmaceutical composition according to claim 8 wherein simvastatin is present in an amount from 5 to 80 mg per tablet.

10. A pharmaceutical composition according to claims 6 or 7 wherein simethicone is present in an amount from 25 to 250 mg per tablet.

11. A pharmaceutical composition according to claim 10 wherein simethicone is present in an amount of 125 mg per tablet.

12. A pharmaceutical composition according to any one of the preceding claims further comprising one or more diluents, one or more binders, one or more disintegrants and one or more lubricants.

13. A pharmaceutical composition according to claim 12 wherein the diluent is selected from the group consisting of microcrystalline celluloses and their derivatives, lactose, mannitol, calcium phosphates, starch, and the mixtures thereof.

14. A pharmaceutical composition according to claims 5 to 12 wherein the binder is selected from the group consisting of starch, polyethylene glycols, polyvinylpyrrolidones, cellulose derivatives, and the mixtures thereof.

15. A pharmaceutical composition according to claim 12 wherein the disintegrant is selected from the group consisting of colloidal silicon dioxide, croscarmellose, la polyvinylpyrrolidone, starch and its pregelatinized derivatives, and the mixtures thereof.

16. A pharmaceutical composition according to claim 12 wherein the lubricant is selected from the group consisting of talc, magnesium stearate, stearic acid, sodium stearyl fumarate, PEG 8000, and the mixtures thereof.

17. A pharmaceutical composition according to any one of the preceding claims further comprising one or more antioxidants and one or more wetting agents.

18. A pharmaceutical composition according to claim 7 wherein the coating of the tablet comprises a cellulose derivative or its pharmaceutically acceptable salt, an acrylic polymer, triethyl citrate, titanium dioxide and one or more lubricants.

19. A pharmaceutical composition according to claim 18 wherein the cellulose derivative is hydroxypropyl methylcellulose or a pharmaceutically acceptable salt thereof.

20. A pharmaceutical composition according to claim 18 or 19 wherein the pharmaceutically acceptable salt is the sodium salt.

21. A pharmaceutical composition according to any one of the preceding claims further comprising one or more colouring agents.

22. A process for preparing a pharmaceutical composition according to any one of the preceding claims by direct compression of components thereof.
